(19) Europäisches Patentamt
European Patent Office
Office européen des brevets

(11) Publication number: **0 090 202**
**B1**

# (12) EUROPEAN PATENT SPECIFICATION

(45) Date of publication of patent specification: **30.12.86**

(51) Int. Cl.$^4$: **C 07 D 317/58**

(21) Application number: **83102198.5**

(22) Date of filing: **06.03.83**

## (54) Process for preparing p.chlorophenoxyacetyl-piperonylpiperazine.

(30) Priority: **29.03.82 IT 2044382**

(43) Date of publication of application:
**05.10.83 Bulletin 83/40**

(45) Publication of the grant of the patent:
**30.12.86 Bulletin 86/52**

(84) Designated Contracting States:
**AT BE CH DE FR GB LI LU NL SE**

(56) References cited:
**FR-A-2 192 820**
**FR-A-2 258 838**
**FR-M- 7 524**

**HOUBEN-WEYL: "Methoden der organischen Chemie", vol. XII/2, part 2, 1964, pages 465-466, Stuttgart, DE., "Phosphorsäure-triamide"**

(73) Proprietor: **RAVIZZA S.p.A.**
**35, Via Europa**
**I-20053 Muggio' (Milano) (IT)**

(72) Inventor: **Mauri, Francesco**
**Via Boito 65**
**Monza, Milan (IT)**

(74) Representative: **Gervasi, Gemma, Dr. et al**
**Studio Brevetti e Marchi NOTARBARTOLO & GERVASI 33, Viale Bianca Maria**
**I-20122 Milano (IT)**

**EP 0 090 202 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to an improved process for preparing the compound p.chlorophenoxyacetyl-piperonylpiperazine ("Fipexide") of formula:

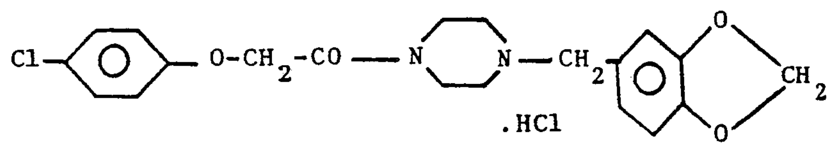

This compound is known for its therapeutic properties based on its antidepressive action and stimulating action on the nervous system, and is also distinguished in comparison with other compounds of the same class and similar therapeutic effect by its very low toxicity, both when administered orally and subcutaneously. Details of the therapeutic use of this compound can be obtained from French medicament patent No. 7524 M.

The process according to the present invention consists of reacting phosphorus oxychloride $POCl_3$ with piperonylpiperazine in an inert solvent, to prepare piperonylpiperazine phosphoramide:

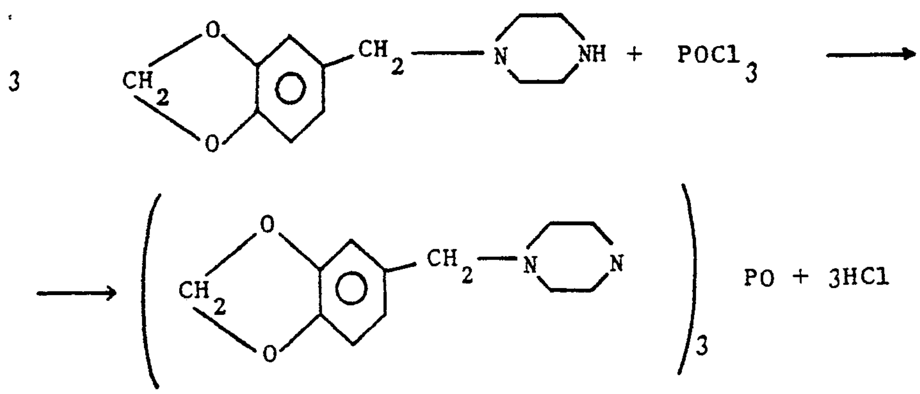

An excess of piperonylpiperazine over the stoichiometric is used, and this serves as an acceptor for the acid which is evolved during said reaction. The piperonylpiperazine hydrochloride formed in this manner is separated from the inert solvent solution containing the phosphoramide. This latter, while still in solution, is reacted with p.chlorophenoxyacetic acid in accordance with the following reaction scheme:

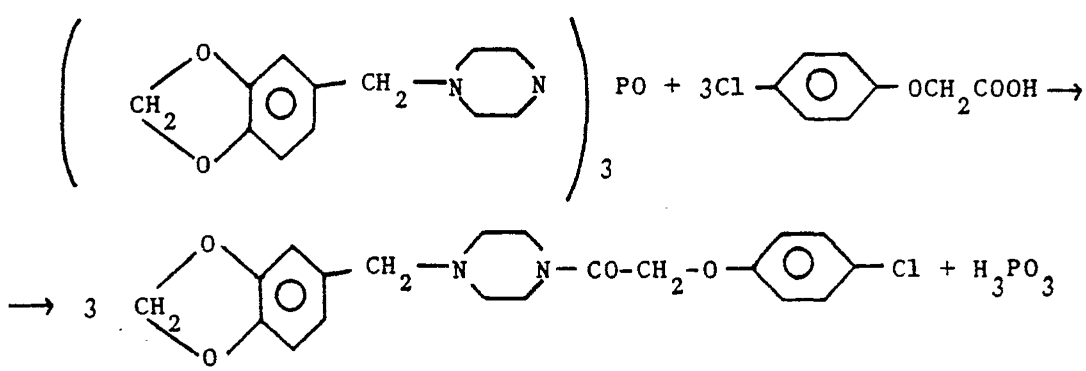

The by-product $H_3PO_3$ can be easily removed by aqueous alkaline wash. The product in question can then be recovered from the solution in its basic form. It can be converted into the corresponding crystalline hydrochloride by adding HCl.

The process according to the invention offers considerable advantages over the known method disclosed in FR—M—7524. In this respect, this latter uses p.chlorophenoxyacetyl chloride which itself must be obtained by chlorinating p.chlorophenoxyacetic acid, for example with $SOCl_2$.

In addition to representing an extra stage, this chlorination requires considerable time, and also poses the problem of eliminating the $SO_2$ and HCl by-products of the reaction.

A further considerable advantage of the process is that the first stage, i.e. the preparation of the phosphoramide, and the subsequent stage of reaction with p.chlorophenoxyacetic acid, can be carried out in the same solvent by adding the acid to the phosphoramide solution.

The example given hereinafter illustrates one embodiment of the invention, but without representing any operational limitation.

Example

55 kg (250 moles) of piperonylpiperazine are dissolved in 130 kg of toluene, the solution is cooled to 0°—5°C, and then 7.5 kg of $POCl_3$ (48.86 moles) dissolved in 12 kg of toluene are added thereto.

The mixture is kept stirred for 2 hours and is allowed to rise to ambient temperature.

The piperonylpiperazine hydrochloride formed in the solid crystalline state is separated by filtration to give a quantity of 32 kg (124.75 moles).

24 kg (129 moles) of p.chlorophenoxyacetic acid are then added to the solution of phosphoramide in toluene.

The mixture is heated to boiling and is kept boiling for 12 hours. An abundant precipitate forms. After cooling, 150 kg of water are added, the mixture is alkalised to pH 10 with 20% NaOH, is stirred for 2 hours and is then centrifuged. 29 kg if "Fipexide" in basic form are obtained. The toluene phase is separated from the mother liquors, is concentrated to a small volume and cooled.

In this manner a further 9 kg of basic "Fipexide" crystallise, and are collected.

The two fractions of Fipexide are dissolved at boiling point in 170 l of a 70% ethanol-$H_2O$ mixture to which 10 l of concentrated HCl have been added, the solution is filtered and cooled on which 36 kg of "Fipexide" crystallise, a further 2 kg of product being obtained by evaporating the mother liquors. Yield 72%.

The base can be recovered from the piperonylpiperazine hydrochloride separated as stated heretofore, by dissolving the hydrochloride in 100 kg of water, and adjusting the pH to 10 with a 20% NaOH solution. It is then extracted 3 times with toluene (45 kg each time). The solution of toluene obtained in this manner can be reused in a subsequent preparation after removing the water azeotropically.

**Claims**

1. A process for preparing p.chlorophenoxyacetyl-piperonylpiperazine, consisting of preparing the phosphoramide of piperonylpiperazine by reacting an excess of this latter with $POCl_3$ in an inert solvent, then reacting the phosphoramide in the same inert solvent with p.chlorophenoxyacetic acid, then eliminating $H_3PO_3$ by aqueous extraction, and then separating the required product from the solution.

2. A process as claimed in claim 1, wherein toluene is used as the reaction solvent.

3. A process as claimed in claim 1, wherein p.chlorophenoxyacetyl-piperonylpiperazine hydrochloride is obtained by adding concentrated HCl to the final solution of the product.

**Patentansprüche**

1. Verfahren zum Herstellen von p-Chlorphenoxyacetylpiperonylpiperazin bestehend im Herstellen des Phosphoramids von Piperonylpiperazin durch Umsetzen eines Überschusses dieses letzteren mit $POCl_3$ in einem inerten Lösungsmittel, im anschließenden Umsetzen des Phosphoramids im gleichen inerten Lösungsmittel mit p-Chlorphenoxyessigsäure, im anschließenden Entfernen von $H_3PO_3$ durch wässerige Extraktion und schließlich im Abtrennen des gewünschten Produktes von der Lösung.

2. Vefahren, wie in Anspruch 1 beansprucht, worin Toluol als das Reaktionslösungsmittel verwendet wird.

3. Verfahren, wie in Anspruch 1 beansprucht, worin p-Chlorphenoxyacetylpiperonylpiperazinhydrochlorid durch Zusetzen von konz.HCl zu der Endlösung des Produktes erhalten wird.

**Revendications**

1. Procédé de préparation de p.chlorophénoxyacétyl-pipéronylpipérazine, consistant à préparer le phosphoramide de pipéronylpipérazine par réaction d'un excès de cette dernière avec $POCl_3$ dans un solvant inerte, puis à faire réagir le phosphoramide dans le même solvant inerte avec l'acide p.chloro-phénoxyacétique, puis à éliminer $H_3PO_3$ par extraction aqueuse et à séparer ensuite le produit voulu de la solution.

2. Procédé selon la revendication 1, dans lequel le toluène est utilisé comme solvant réactionnel.

3. Procédé selon la revendication 1, dans lequel le chlorhydrate de p.chlorophénoxyacétyl-pipéronylpipérazine est obtenu par addition de HCl concentré à la solution finale du produit.